Europäisches Patentamt

European Patent Office

Office européen des brevets

(11) Veröffentlichungsnummer: **0 379 599**

**A1**

# (12) EUROPÄISCHE PATENTANMELDUNG

(21) Anmeldenummer: 89101041.5

(22) Anmeldetag: 21.01.89

(51) Int. Cl.5: **C08L 97/02, B01J 20/24, A61K 9/20**

(43) Veröffentlichungstag der Anmeldung:
01.08.90 Patentblatt 90/31

(84) Benannte Vertragsstaaten:
AT BE CH DE FR GB IT LI NL

(71) Anmelder: **FIRMA CELCOMMERZ HIGH-CHEM.-PRODUKTE GMBH & CO. KG**
**Fleenerweg 2**
**D-4050 Mönchengladbach 1(DE)**

(72) Erfinder: **Rettenmaier, Felix**
**Fleenerweg 2**
**D-4050 Mönchengladbach(DE)**

(74) Vertreter: **Sroka, Peter-Christian, Dipl.-Ing. et al**
**Dominikanerstrasse 37 Postfach 111038**
**D-4000 Düsseldorf 11(DE)**

(54) **Celluloserohprodukt zur Verwendung als Füllmittel, Filter- und Presshilfsmittel, Sprengmittel sowie Reaktionsmittel-Trägerstoff.**

(57) Ein Celluloserohprodukt zur Verwendung als Füllmittel, Filter- und Preßhilfsmittel, Sprengmittel sowie Reaktionsmittel-Trägerstoff besteht erfindungsgemäß aus mechanisch auf eine Partikelgröße von 0-2000 $\mu$m, vorzugsweise 0-400 $\mu$m, zerkleinerten Einjahrespflanzen, vorzugsweise Stroh.

EP 0 379 599 A1

## Celluloserohprodukt zur Verwendung als Füllmittel, Filter- und Preßhilfsmittel, Sprengmittel sowie Reaktionsmittel-Trägerstoff

In den zurückliegenden Jahren haben Füllstoffe sowie Filter- und Preßhilfsmittel, die aus hochwertigen Rohstoffen wie Holz-Cellulose hergestellt sind, einen besonderen, meistens negativen Stellenwert erhalten, weil durch die Verwertung von Bäumen zu Holz-Cellulose-Füllstoffen ein übermäßiger Eingriff in die Ökologie der Natur vorgenommen wird.

Diese negativen Begleiterscheinungen werden ausgeschaltet mit dem erfindungsgemäßen Celluloserohprodukt zur Verwendung als Füllmittel, Filter-und Preßhilfsmittel, Sprengmittel sowie Reaktionsmittel-Trägerstoff, welches dadurch gekennzeichnet ist, daß es aus mechanisch auf eine Partikelgröße von 0-2000 $\mu$m, vorzugsweise 0-400 $\mu$m, zerkleinerten Einjahrespflanzen, vorzugsweise Stroh, zum Beispiel Weizenstroh, Gerstenstroh, Haferstroh, Roggenstroh, Reisstroh, Maisstroh, Hirsestroh usw. besteht.

Stroh steht in ausreichend großen Mengen in allen Kontinenten zur Verfügung. Es ist ein alljährlich automatisch anfallendes Nebenprodukt aus der Getreideherstellung und somit ohne hochwertigen ökologischen Effekt. Im Gegenteil, die Landwirte haben für Stroh häufig keine Verwertung, da die Viehhaltungen aus Rationalisierungsgründen ohne Stroh arbeiten. Somit wird Stroh vielfach durch Abbrennen vernichtet, was weder volkswirtschaftlich noch im Sinne des Umweltschutzes sinnvoll und richtig ist.

Bekannt ist eine industrielle Nutzung von Stroh zur Gewinnung von sogenannter Sulfit- oder Sulfatcellulose. Ausgangsprodukt ist aber gewöhnliches, unaufgeschlossenes Stroh, das durch eine chemische Behandlung verändert wird. Bei der Erfindung handelt es sich hingegen um mechanisch aufbereitete Einjahrespflanzen - Stroh - zu Mehlen und feinen Fasern, die entsprechende Eigenschaften aufweisen. Diese Produkte sind neu.

Der Aufbau eines Strohhalmes ist von fast gleichartiger Faserstruktur. Die Fasern sind teilweise von unterschiedlicher Länge, jedoch immer in gleicher Richtung geordnet.

Wird diese Struktur zerkleinert, z.B. mit Kalandern oder anderen Zerkleinerungswerkzeugen wie Schlag- und/oder Schneidmühlen, erreicht man ein faseriges Pulver. Dieses Pulver wird mittels Siebmaschinen klassifiziert.

Das mechanisch aufgeschlossene, schlanke erfindungsgemäße Produkt aus Einjahrespflanzen, insbesondere Stroh, und mit seiner zerklüfteten Struktur ist ein idealer Füllstoff mit guten Armierungseigenschaften. Durch die Auswahl der Partikelfeinheit entstehen mehr oder weniger schlanke und in der Länge definierte Fasern. Dadurch sind die gewünschten Effekte einstellbar.

Die Faserstruktur von Stroh-Pulver läßt sich deutlich verbessern, wenn das vorbereitete Stroh vor der Aufarbeitung mit Flüssigkeit angereichert wird, z.B. durch Benetzen und/oder Bedampfen mit Wasser, Lösungsmitteln etc. .

Sinn der Erfindung ist es, aus Rohfasern von Einjahrespflanzen für die Industrie einen geeigneten, funktionellen Füllstoff sowie Filterhilfsmittel zu fertigen. Dieses Produkt erbringt im vollem Umfang die Qualitäten von bisher üblichen, organischen Füllstoffen für die möglichen Einsatzbereiche, ohne dabei ökologische Eingriffe zu verursachen.

Stroh läßt sich mit dafür geeigneten Maschinen in diverse Feinheiten und Faserstrukturen aufschließen. Diese organischen Rohfaser-Füllstoffe kommen in der chemischen Industrie zum Einsatz.

Als Stroh-Rohfaser-Füllstoff wird eine Korngröße von 0-2000 $\mu$m, vorzugsweise 0-200 $\mu$m, insbesondere 0-90 $\mu$m, bevorzugt. Das Einsatzgebiet für diese funktionellen Füllstoffe ist die Fertigung von Duroplast- und Thermoplast-Kunststoffen, Linoleum-Bodenbeläge und ähnliches.

Bedingt durch die offenporige Faserstruktur der Rohfaser von Einjahrespflanzen läßt sich eine bis ins Extrem gehende Trocknung durchführen.

Erfindungsgemäß lassen sich dadurch vorzugsweise thermoplastische Kunststoffe mit Stroh-Rohfaser-Füllstoffen füllen.

Durch die Mikroporösität der Stroh-Rohfaser entsteht bei der Zerkleinerung eine große Oberfläche. Durch diese Zerklüftungen entstehen Widerstände, bilden sich Beruhigungszonen und Ablagerungsmöglichkeiten.

Erfindungsgemäß wird diese Gegebenheit bei der Verwendung von Stroh-Rohfaser-Pulver bei der Filtrationstechnik nutzbar. Gelöst wird diese Aufgabe erfindungsgemäß durch die Verwendung von Stroh-Rohfaser-Filterhilfsstoff mit einer Korngröße von 0-2000 $\mu$m, vorzugsweise 0-160 $\mu$m.

Der Einsatzbereich ist die Filtration (Trennen von Festteilen aus Flüssigkeiten) uner Zuhilfenahme von geeigneten Filtermaschinen (Anschwemmfilter). Bei den zu filtrierenden Medien kann es sich um Produkte des Lebensmittelbereiches (z.B. Speiseöl), um Produkte des chemischen Bereiches (z.B. Titandioxyd) oder um Produkte des Abwassersektors handeln.

2

Unter den Bereich organischer Filterhilfsmittel auf Strohbasis fallen auch Mischungen mit anderen organischen und anorganischen Filterhilfsmitteln, wobei der Anteil an Stroh-Filterhilfstoff z.B. 50 % bezogen auf Volumen und/oder Gewicht, betragen kann.

Durch geeignete, mechanische Aufschlußverfahren läßt sich die Faser von Einjahrespflanzen - vorzugsweise Stroh - so auflösen, daß feine, in Längsrichtung (Faserrichtung) zerlegte, feinporige Partikel entstehen. Diese erfindungsgemäße Stroh-Faser ist das Austauschprodukt zu Holzschliff, das vorzugsweise zur Herstellung von Zeitungspapier Verwendung findet.

Der erfindungsgemäß Füllstoff aus Einjahrespflanzen - vorzugsweise Stroh - hat durch sein bei der Aufarbeitung aufgeschlossenes Kapillarsystem eine ausgezeichnete Sprengwirkung, z.B. im Sinne von sogenannten Tablettensprengmitteln. So zerfallen z.B. chemische Substanzen und/oder Wirkstoffe, die mit Stroh-Füllstoff - vorzugsweise in der Feinheit 0-160 μm - hergestellt werden (z.B. Presslinge, Granulate oder ähnliches) rasch (innerhalb weniger Sekunden), wenn sie mit Flüssigkeit (z.B. Wasser) in Verbindung gebracht werden.

Stroh-Rohfasern, aufgeschlossen auf eine Feinheit von 0-2000 μm, vorzugsweise 0-400 μm, eignen sich erfindungsgemäß aufgrund der mikroporösen Faserstruktur vorzüglich als Presshilfsmittel für fett-, schleim- und pektinhaltige Produkte, zum Beispiel
- zum Separieren von Organsubstanzen im Pharmabereich
- zum Separieren von schleimhaltigen Grundsubstanzen im chemischen Bereich
- zum Separieren von Pektinen aus Früchten und Fruchttrub.

Durch die Molekularstruktur der aufgeschlossenen Stroh-Rohfaser - vorzugsweise im Feinheitsbereich von 0-200 μm -, läßt sich der erfindungsgemäße funktionelle Stroh-Füllstoff problemlos mit Antioxydantien, Pestiziden, Fungiziden, Silanen, Stearaten, zwei- und höherwertigen Alkoholen oder anderen Fest/Flüssig-Stoffen benetzen und/oder ummanteln (coaten). Für die Benetzung und/oder Coatung reichen im allgemeinen Mengen zwischen 0,1-10 %, vorzugsweise 0,5-3 Gewichtsprozent, aus. Es können jedoch in speziellen Fällen auch größere oder geringere Mengen zweckmäßig sein. Größere Mengen von Flammschutzmitteln bewirken bei der Stroh-Rohfaser eine schwere bis schwerste Entflammbarkeit. Dieser erfindungsgemäße funktionelle Stroh-Füllstoff eignet sich vorzugsweise zur Herstellung bauchemischer Produkte, zum Beispiel
- als Füllstoff zur Herstellung von Fertigputzen für Innen und Außen
- als Füllstoff zur Herstellung von Spachtelmassen
- als Isolierfüllstoff in Gipskarton-Bauplatten
- als Füllstoff zur Herstellung von Antidröhnmassen und als Brandschutzbeschichtung
- als Füllstoff bituminöser Produkte.

Der Zusatz von Antioxydantien, Pestiziden, Fungiziden, Silanen, Stearaten, zwei- oder höherwertigen Alkoholen, Flammschutzmittel oder sonstige organische Produkte sowie Chemikalien erfolgt vorzugsweise am besten während des Zerkleinerungsvorganges in der Trockenfaser. Die Umhüllung (Coatung) ist auch in einem zweiten oder dritten Arbeitsgang möglich. Durch die Behandlung wird die Oberflächenstruktur üblicherweise verändert, so daß sich Veränderungen im Schüttgewicht und im Fließverhalten ergeben.

Der erfindungsgemäße funktionelle Stroh-Füllstoff läßt sich durch das im Zerkleinerungsaufschluß offengelegte Poren-Faser-Kapillarnetz rasch und vollständig mit chemischen wie auch organischen Farbstoffen tränken und durchfärben. Die mikroporöse Struktur des Stroh-Füllstoffes bleibt dadurch weitestgehend erhalten. In gleicher Weise wie ein Durchfärben möglich ist, läßt sich auch ein Bleichvorgang und somit optische Aufhellung des erfindungsgemäßen Stroh-Füllstoffes durchführen, ohne daß die Faserstruktur zerstört wird. Als Bleichmittel lassen sich herkömmliche, chemische Mittel wie auch moderne Sauerstoff-Bleichvorgänge anwenden.

Je nach Herkunft der Einjahrespflanzen weisen die daraus hergestellten funktionellen Füllstoffe eine unterschiedliche arteigene Farbe aus. Dieses geht von hellem gelb bis zu einer grau-braunen Farbe. Die Farbe einer gesunden und trockenen Einjahrespflanze hat keinen Einfluß auf die Qualität, den Aufschlußvorgang, die Mikroporösität der Faserstruktur und des Kapillarnetzes des erfindungsgemäßen funktionellen organischen Füllstoffes aus Einjahrespflanzen, vorzugsweise Stroh.

Die Einsatzmengen des erfindungsgemäßen Füllstoffes aus Einjahrespflanzen - vorzugsweise Stroh - in den Feinheitsbereichen 0-90 μm / 0-150 μm / 0-160 μm / 0-400 μm richten sich jeweils für die vorgenannten Bereiche nach den Mengen der anderen Zuschlagstoffen und den zu erreichenden Effekten. Das Spektrum reicht von 0,5 Gew.% bis 80 Gew. %. Der erfindungsgemäße Stroh-Füllstoff ist dann in der Lage, dem daraus hergestellten Produkt "Körper" zu verleihen, wenn der Sättigungsgrad erreicht wird. Diese Füllstoffoptimierung ist nur mit dem erfindungsgemäßen mikroporösen Stroh-Füllstoff erreichbar.

Die durchschnittlichen analythischen Werte von aufgeschlossenen, funktionellen Füllstoffen und Filterhilfsmitteln aus Einjahrespflanzen, wie erfindungsgemäß beschrieben, sind:

3

| Organische Substanz: | ca. 96 % |
|---|---|
| Mineralische Bestandteile: | ca. 3 % |
| Spurenelemente: | ca. 1 % |

**Ansprüche**

1. Celluloserohprodukt zur Verwendung als Füllmittel, Filter- und Preßhilfsmittel, Sprengmittel sowie Reaktionsmittel-Trägerstoff, dadurch gekennzeichnet, daß es aus mechanisch auf eine Partikelgröße von 0-2000 µm, vorzugsweise 0-400 µm, zerkleinerten Einjahrespflanzen, vorzugsweise Stroh, besteht.

2. Celluloserohprodukt nach Anspruch 1, dadurch gekennzeichnet, daß es aus vor der mechanischen Zerkleinerung mit einer Flüssigkeit benetztem oder bedampftem Stroh hergestellt ist.

3. Celluloseprodukt zur Verwendung als Füllmittel nach Anspruch 1, dadurch gekennzeichnet, daß es auf eine Partikelgröße von 0-200 µm, vorzugsweise 0-90 µm, zerkleinert ist.

4. Celluloserohprodukt als Filtermittel und als Sprengmittel nach Anspruch 1, dadurch gekennzeichnet, daß es auf eine Partikelgröße von 0-200 µm, vorzugsweise 0-160 µm, zerkleinert ist.

5. Celluloserohprodukt zur Verwendung als Preßhilfsmittel nach Anspruch 1, dadurch gekennzeichnet, daß es auf eine Partikelgröße von 0-400 µm zerkleinert ist.

6. Celluloseprodukt zur Verwendung als Reaktionsmittel-Trägerstoff nach Anspruch 1, dadurch gekennzeichnet, daß es auf eine Partikelgröße von 0-200 µm zerkleinert und mit Reaktionsmitteln wie Antioxydantien, Pestiziden, Fungiziden, Silanen, Stearaten, zwei- und höherwertigen Alkoholen oder anderen Fest-Flüssig-Stoffen benetzt und/oder beschichtet ist.

7. Celluloserohprodukt nach Anspruch 6, dadurch gekennzeichnet, daß es, bezogen auf das Gesamtgewicht, mit 0,1-10 Gewichtsprozent, vorzugsweise 0,5-3 Gewichtsprozent, Reaktionsmittel benetzt und/oder beschichtet ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | FR-A-1 051 196 (E.A. KROIS) <br> * Zusammenfassung; Seite 1, rechte Spalte, letzter Absatz - Seite 2, linke Spalte, erster Absatz * <br> --- | 1,3,5 | C 08 L 97/02 <br> B 01 J 20/24 <br> A 61 K 9/20 |
| X | CHEMICAL ABSTRACTS, Band 96, Nr. 13, 29. März 1982, Seite 557, Zusammenfassung Nr. 102445y, Columbus, Ohio, US; E.L. AGABEKYAN et al.: "Effect of straw dispersity on cellulolytic activity of the yeast Aspergillus terreus 17P", & PRIKL. BIOKHIM. MIKROBIOL. 1981, 17(6), 859-63 <br> * Zusammenfassung * <br> --- | 1 | |
| X | NL-B- 81 793 (SCHOLTEN'S AARDAPPELMEELFABRIEKEN) <br> * Insgesamt * <br> ----- | 1 | |

RECHERCHIERTE SACHGEBIETE (Int. Cl.5)

C 08 L
A 61 K

Der vorliegende Recherchenbericht wurde für alle Patentansprüche erstellt

| Recherchenort | Abschlußdatum der Recherche | Prüfer |
|---|---|---|
| DEN HAAG | 05-10-1989 | LENSEN H.W.M. |

KATEGORIE DER GENANNTEN DOKUMENTE

X : von besonderer Bedeutung allein betrachtet
Y : von besonderer Bedeutung in Verbindung mit einer
  anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
  nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes
  Dokument

EPO FORM 1503 03.82 (P0403)